# FASCICULE DE BREVET EUROPEEN

(11) **EP 1 733 696 B1**
(45) Date de publication et mention de la délivrance du brevet: **16.04.2008**
(21) Numéro de dépôt: 05012853.7
(22) Date de dépôt: 15.06.2005
(51) Int. Cl.: A61C 1/14, A61B 17/16

(54) **Pièce à main à usage dentaire ou chirurgical comportant une pince élastique**
Handstück für zahnärztliche oder chirurgische Anwendungen mit einer elastischen Spannzange
Handpiece for dental or surgical use with an elastic collet

(43) Date de publication de la demande: 20.12.2006
(73) Titulaire: Bien-Air Holding SA, 2500 Bienne 6 (CH)
(72) Inventeur: Maître, Luc, 2885 Epauvillers (CH); Ryser, Cyril, 2720 Tramelan (CH)
(74) Mandataire: Laurent, Jean

(56) Documents cités:
- EP-A- 0 505 599
- DE-A1- 2 905 484

## Description

### Arrière-plan de l'invention

La présente invention concerne une pièce à main à usage dentaire ou chirurgical, comportant un arbre creux associé à des moyens d'entraînement en rotation et dans lequel sont montés une pince pour le serrage de la queue d'un outil amovible, une douille de desserrage et un poussoir coulissant placé dans une extrémité arrière de l'arbre creux et commandé par un bouton manuel pour rapprocher mutuellement la pince et la douille de desserrage, la pince comportant une partie annulaire et des mâchoires agencées pour serrer par élasticité la queue de l'outil dans une position de service, ces mâchoires s'étendant longitudinalement à partir de la partie annulaire en direction de la douille de desserrage, laquelle comporte des surfaces extérieures obliques capables de s'appuyer contre des extrémités libres des mâchoires pour les écarter.

Dans la plupart des cas, les pièces à main de ce genre sont entraînées par une turbine à air et tournent à des vitesses très élevées, jusqu'à plus de 400'000 tours/minute, ce qui pose des exigences élevées pour le centrage de l'outil par rapport à l'axe de rotation. Pour assurer une retenue suffisante de l'outil pendant le travail, la pince élastique doit résister à une force d'arrachage normalisée d'au moins 22 N sur l'outil. D'autre part, l'opérateur doit changer d'outil fréquemment ; cette opération doit donc être aisée et induire le moins possible d'usure.

Dans une pièce à main dentaire typique de ce genre, telle que décrite par exemple dans les brevets EP 273 259 et EP 505 599, la pince est fixe dans l'arbre creux, sa partie annulaire se trouve du côté de l'outil, c'est-à-dire à l'extrémité avant de l'arbre, et ses mâchoires s'étendent axialement en direction de l'arrière, où la douille de desserrage peut coulisser dans l'arbre creux sous l'effet d'un poussoir manuel lié au couvercle arrière mobile de la pièce à main. La douille de desserrage comporte à l'avant un cône extérieur muni de surfaces d'appui ayant un angle d'ouverture plus fort que les surfaces opposées du cône intérieur des mâchoires de la pince, si bien que le contact entre les deux cônes se fait sur une ligne qui ne change pas de place sur chaque mâchoire pendant la course du poussoir.

Avec la construction selon le brevet EP 273 259, la partie annulaire de la pince assure le centrage de la queue de l'outil à l'extrémité avant de la partie rotative, tandis que l'extrémité arrière de la queue est centrée à l'intérieur de la douille de desserrage. Comme cette pièce est coulissante, elle a nécessairement un léger jeu radial et le centrage n'est donc pas excellent. De plus, la fabrication et l'équilibrage de la pièce formant à la fois la pince et un canon de guidage sont des opérations difficiles et coûteuses. Un autre inconvénient de cet agencement réside dans la longueur totale de l'ensemble comprenant la pince, ladite douille et le poussoir manuel. Cette longueur impose une certaine hauteur de la tête de la pièce à main, hauteur qui est gênante puisqu'il s'agit de la partie que le dentiste doit introduire dans la bouche du patient.

Le brevet EP 505 599 prévoit d'améliorer cette construction au moyen d'un canon de guidage à l'avant et d'un guide fixe disposé dans l'extrémité arrière de l'arbre creux, assurant ainsi un meilleur centrage de la queue de l'outil et comportant à l'intérieur une butée fixe pour la queue de l'outil, mais la longueur de l'ensemble s'en trouve accrue. En outre, cet agencement présente un autre inconvénient par le fait que la douille de desserrage peut se bloquer par friction dans la pince ouverte, sans qu'on puisse la débloquer par insertion de la queue de l'outil, puisque la butée axiale n'est pas sur cette douille.

### Résumé de l'invention

La présente invention a pour objet un nouvel agencement des éléments contenus dans l'arbre creux, notamment de la pince et de ses moyens de commande, assurant à la fois un centrage de haute précision, un fonctionnement fiable et surtout une construction de longueur réduite, permettant de réaliser la tête de la pièce à main sous une forme particulièrement compacte.

A cet effet, il est prévu une pièce à main du genre spécifié en préambule ci-dessus, caractérisée en ce que la douille de desserrage est montée dans une extrémité avant de l'arbre creux, c'est-à-dire du côté de l'outil, et en ce que la pince est montée de manière coulissante dans l'arbre, ses mâchoires étant dirigées vers l'avant, la pince étant liée longitudinalement au poussoir, lequel permet à un opérateur de déplacer la pince en direction de l'avant pour l'appuyer contre la douille de desserrage. Celle-ci peut ainsi être fixe dans l'arbre creux et former un canon de guidage avant pour la queue de l'outil. Selon un mode de réalisation assurant un excellent guidage de l'extrémité arrière de la queue, un canon de guidage arrière pourvu d'un alésage cylindrique est monté de manière fixe dans l'arbre creux, entre la partie annulaire de la pince et la paroi transversale du poussoir de commande, et ce canon comporte des canaux pour des bras longitudinaux reliant le poussoir à la pince.

Grâce à ces dispositions, il est possible de réduire la longueur de l'ensemble rotatif par rapport à l'art antérieur, donc d'obtenir une pièce à main dont la tête a une hauteur aussi faible que possible, sans faire de concessions sur la précision du centrage de l'outil. Les éléments contribuant à cela sont notamment le fait que la double fonction de la douille de desserrage fixe servant aussi de canon de guidage permet de gagner une pièce sur la longueur de l'arbre, et le fait que l'extrémité de la queue de l'outil peut aller presque jusqu'à l'extrémité arrière de l'arbre.

Comme la pince est mobile en direction axiale, une éventuelle traction s'exerçant sur l'outil va tirer la pince vers l'avant en direction de la douille de desserrage et pourrait ainsi faire ouvrir la pince et relâcher l'outil même si la traction était inférieure à la limite normalisée de 22 N. Afin d'éviter une telle ouverture intempestive, il est prévu avantageusement que l'ensemble formé par la pince et le poussoir de commande soit pourvu d'un dispositif de retenue à déclic, agencé pour opposer une résistance initiale prédéterminée à un déplacement de cet ensemble vers l'avant à partir de la position de service. Ce dispositif comporte des moyens d'accrochage élastiques qui prennent appui sur l'arbre creux et qui peuvent être réalisés de différentes façons, en opposant initialement une résistance au déplacement de la pince et du poussoir qui excède sensiblement la limite de 22 N. C'est aussi cette résistance que l'opérateur doit surmonter initialement quand il presse le bouton d'ouverture de la pince pour retirer l'outil. Par contre, dès que le déclic s'est produit et que la pince a commencé à avancer, la résistance opposée par le bouton résulte seulement de la composante axiale des forces exercées par la bague de desserrage sur la pince et peut donc être inférieure à la force initiale. Autrement dit, après le déclic, l'opérateur exerce une force réduite sur le bouton, ce qui améliore le confort d'utilisation de la pièce à main.

### Description sommaire des dessins

D'autres caractéristiques et avantages de la présente invention ressortiront de la description suivante, qui présente un mode de réalisation préféré et diverses variantes à titre d'exemples non limitatifs en se référant aux dessins annexés, dans lesquels :
- la figure 1 est une vue schématique en coupe axiale de la tête d'une pièce à main dentaire à turbine selon un premier mode de réalisation de l'invention,
- la figure 2 est une vue en perspective de la pince visible dans la figure 1,
- la figure 3 est une vue en perspective de la douille de desserrage visible dans la figure 1,
- la figure 4 est une vue en coupe axiale de la douille de la figure 3,
- la figure 5 est une vue en coupe axiale d'une partie de la pince de la figure 2,
- la figure 6 est une vue en perspective d'un canon de guidage arrière visible dans la figure 1,
- la figure 7 est une vue analogue à la figure 3 et représente une variante de la douille de desserrage,
- la figure 8 est une vue schématique en coupe axiale représentant un autre mode de réalisation de l'invention, et
- la figure 9 est une vue en perspective d'un poussoir visible dans la figure 8.

### Description détaillée de divers modes de réalisation

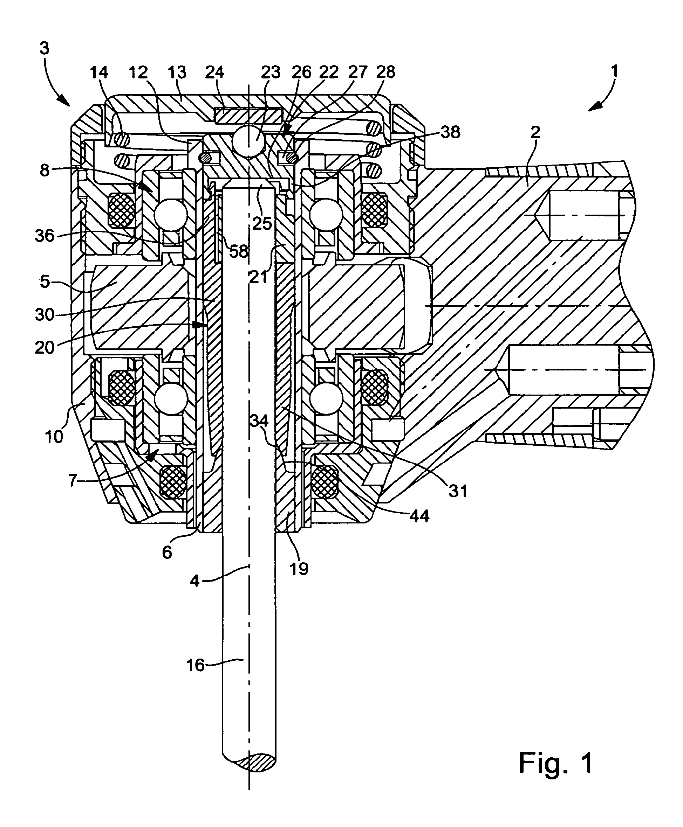

La pièce à main 1 représentée partiellement dans la figure 1 est du type à turbine à air, comportant un manche 2 et une tête 3 à axe 4 approximativement perpendiculaire au manche. Le rotor 5 de la turbine est fixé à un arbre creux 6 supporté par deux paliers 7 et 8 dans le corps 10 de la tête 3, pour tourner autour de l'axe 4. L'arbre 6 a la forme d'un tube ayant une extrémité arrière 12 plus épaisse, pour s'appuyer axialement contre le palier arrière 8. Comme d'habitude, la tête de la pièce à main est fermée à l'arrière, c'est-à-dire en haut dans la position représentée en figure 1, par un couvercle mobile repoussé en butée vers l'arrière par un ressort de rappel 14 et formant un bouton 13 de commande manuelle. Pour tenir de manière amovible la queue cylindrique 16 d'un outil dentaire dans une position centrée sur l'axe 4, l'arbre 6 contient, de l'avant à l'arrière, une douille de desserrage 19, une pince élastique 20, un canon de guidage arrière 21 et un poussoir 22. Ce dernier porte sur sa face arrière une bille 23 contre laquelle une plaquette dure 24 du bouton 13 peut s'appuyer lorsqu'on presse ce bouton. Les éléments 19, 20 et 21 ont un alésage central qui reçoit la queue 16 de l'outil, tandis que l'extrémité arrière 25 de la queue bute contre une paroi transversale 26 du poussoir 22. Une rainure annulaire 27 est ménagée dans le pourtour du poussoir 22 pour loger un ressort-fil 28 qui est comprimé radialement par l'arbre 6 et s'accroche par encliquetage dans une petite gorge intérieure de cet arbre, formant ainsi un dispositif de retenue à déclic qui maintient axialement le poussoir 22 en position normale.

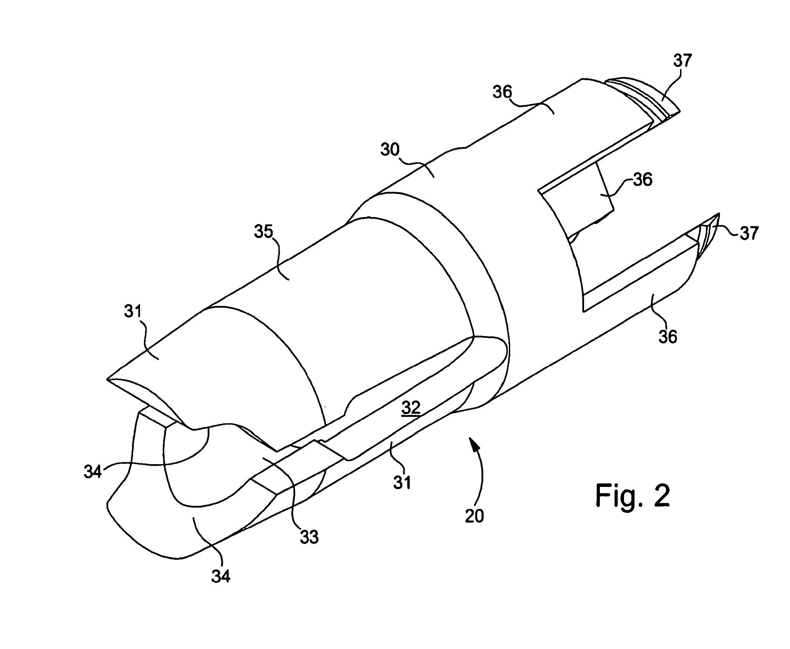

La pince 20 est une construction monobloc, représentée dans la figure 2, comportant une partie annulaire 30 dont la surface extérieure cylindrique s'ajuste de manière coulissante dans l'arbre creux 6. A partir de cette partie annulaire s'étendent vers l'avant des mâchoires élastiques 31, qui sont ici au nombre de deux et sont séparées par des fentes longitudinales 32. Dans la partie avant de chaque mâchoire 31 se trouve une surface interne de forme sensiblement cylindrique, formant un mors 33 pour serrer la queue 16 de l'outil. En avant du mors 33, la face intérieure de la mâchoire est échancrée pour former une surface oblique 34, inclinée longitudinalement et utilisée pour écarter les mâchoires. La partie arrière 35 de chaque mâchoire est amincie afin d'être flexible lorsqu'on exerce une force à composante radiale sur la surface oblique 34.

Au repos, l'espace compris entre les mors 33 des mâchoires a un diamètre plus petit que le diamètre standard de la queue 16 des outils à utiliser, de sorte que les mâchoires serrent élastiquement cette queue lorsqu'elle est placée entre elles. Les mâchoires sont dimensionnées de façon que la force de serrage et la friction en résultant soient suffisantes pour opposer une résistance à l'arrachement de l'outil qui soit supérieure à la limite normalisée.

L'arrière de la pince 20 comprend des bras longitudinaux 36 flexibles radialement, ici au nombre de trois, qui s'étendent à partir de la partie annulaire 30 et dont l'extrémité libre 37 présente un profil d'accrochage sur la face extérieure. Ces bras 36 ont pour fonction d'accrocher la pince 20 au poussoir 22. Celui-ci comporte pour cela, comme on le voit dans la figure 1, un rebord annulaire 38 qui s'étend en avant de la paroi transversale 26 et présente à l'intérieur un profil d'accrochage complémentaire à celui des bras de la pince. En outre, le rebord 38 bute axialement contre les bras 36 pour pouvoir pousser la pince vers l'avant. Les deux profils d'accrochage et la flexibilité des bras 36 sont combinés pour assurer la retenue longitudinale prescrite de la pince dans toutes les conditions d'utilisation de la pièce à main, mais permettre néanmoins une séparation au moyen d'une force plus grande afin de permettre le démontage. L'extrémité 37 de chaque bras 36 comporte un chanfrein permettant d'assembler à nouveau les deux pièces par encliquetage grâce à la flexibilité des bras 36.

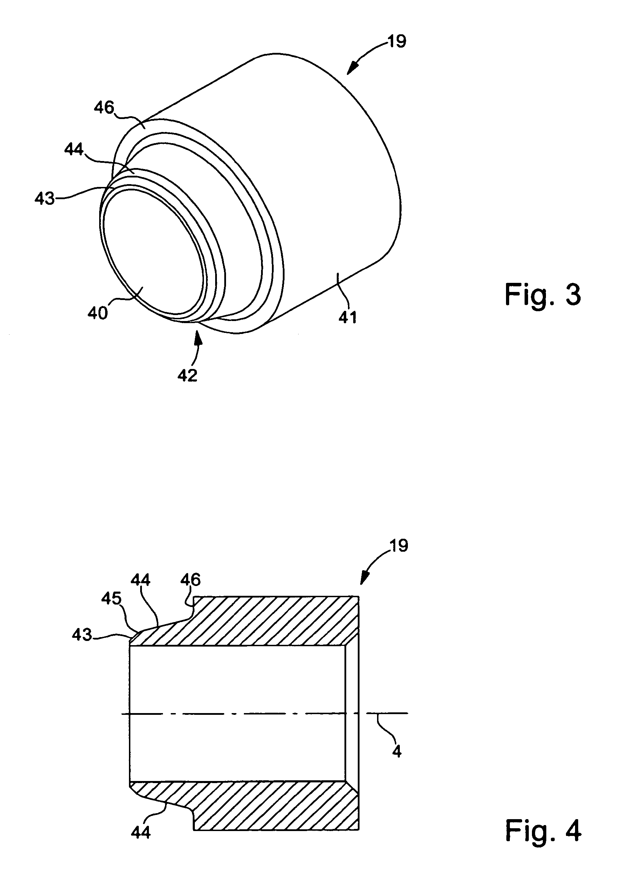

Une forme de réalisation de la douille de desserrage 19 est représentée en détail dans les figures 3 et 4. Son alésage intérieur 40 est calibré pour recevoir la queue 16 de l'outil, de sorte que la douille 19 constitue un canon de guidage avant. Sa surface extérieure 41 est cylindrique et s'ajuste sans jeu dans l'arbre creux 6, auquel la douille 19 est de préférence soudée. A l'avant, la douille 19 comporte un cône extérieur 42 comprenant deux parties dont les angles de conicité sont différents, formant une première surface oblique 43 dont l'angle de conicité par rapport à l'axe 4 est plus grand que celui de la seconde surface oblique 44. Les surfaces 43 et 44 sont raccordées par une surface arrondie 45. Derrière la surface 44 se trouve une surface transversale 46. Les surfaces 43 à 46 de la douille sont destinées à coopérer avec l'extrémité de chaque mâchoire de la pince 20.

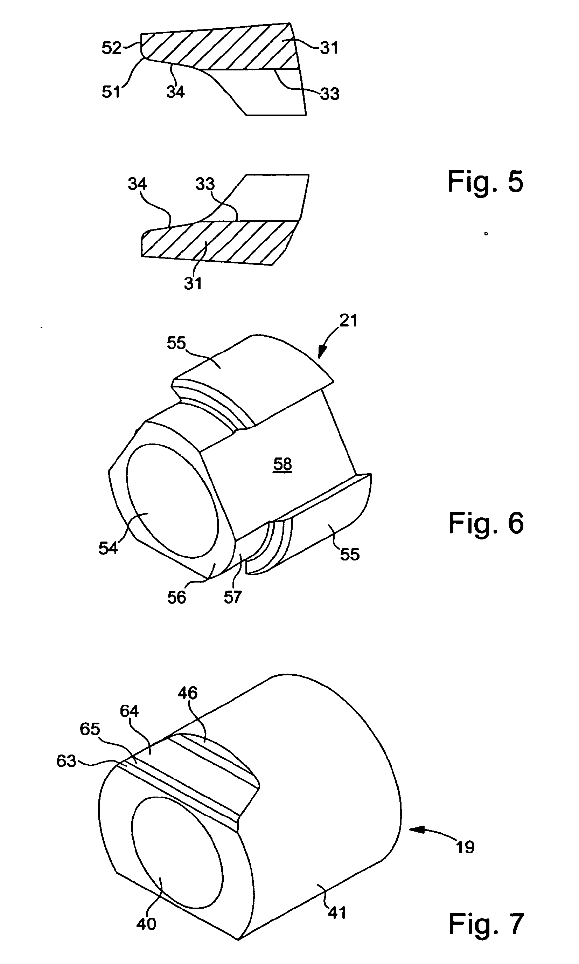

La figure 5 est une vue en coupe agrandie des extrémités libres des mâchoires 31 de la pince 20 représentée dans les figures 1 et 2. Sur la face intérieure de la mâchoire, la surface du mors 33 est précédée par la surface oblique 34 qui est sensiblement plane dans cet exemple, mais pourrait aussi avoir une configuration conique dans d'autres formes de réalisation. A l'avant, la surface 34 est raccordée par une surface arrondie 51 à une surface frontale 52 de la mâchoire. Les surfaces 34 et 51 de la mâchoire sont destinées à s'appuyer contre la surface oblique 44 de la douille de desserrage 19 pour écarter les mâchoires 31 de la pince. La surface frontale 52 peut buter contre la surface transversale 46 de la douille 19 pour arrêter la course de la pince en position ouverte.

La figure 6 montre la forme du canon de guidage arrière 21, comportant un alésage cylindrique 54 dans lequel la queue 16 de l'outil est guidée avec précision. Le canon 21 a une surface périphérique 55 de forme cylindrique qui s'ajuste dans l'arbre creux 6, auquel il est fixé par exemple par soudage. Entre cette surface et l'extrémité arrière 56 du canon 21 se trouve un tronçon 57 de plus petit diamètre, pour laisser place au rebord 38 du poussoir 22. La surface cylindrique 55 est interrompue par trois rainures longitudinales formant des canaux 58 dans lesquels les bras élastiques 36 de la pince passent avec un certain jeu radial, comme on le voit dans la figure 1, afin de pouvoir fléchir vers l'intérieur pour s'encliqueter sur le poussoir 22. La pince est solidarisée en rotation avec l'arbre 6 par ses bras 36 emboîtés dans les canaux 38.

Au cours de la fabrication de la pièce à main, la présence des deux canons de guidage fixes 19 (c'est-à-dire la douille de serrage) et 21 fixés dans l'arbre creux 6 permet avantageusement une opération de rectification des surfaces extérieures de l'arbre 6, en installant l'arbre avec ces deux canons de guidage sur une tige de référence occupant la place de la queue 16 de l'outil. Ceci permet de rectifier les portées des paliers 7 et 8 sur l'arbre afin d'assurer leur parfaite concentricité avec les alésages des deux canons de guidage.

L'agencement qui vient d'être décrit fonctionne de la manière suivante. Dans la position de service qu'on voit dans la figure 1, la pince 20 ne s'appuie pas sur la douille de desserrage 19. Cette douille, au lieu d'être soudée à l'arbre, pourrait être montée de manière réglable longitudinalement afin de régler un espace entre elle et la pince. La queue 16 de l'outil dentaire est guidée latéralement par les deux canons 19 et 21, bute axialement contre le poussoir 22 à l'arrière et reste maintenue par les mors de la pince 20 contre une force d'arrachement ne dépassant pas la limite normalisée. Cette force est transmise par les bras 36 de la pince au poussoir 22, puis par le ressort 28 à l'arbre 6 et aux paliers 7 et 8. La bille 23 ne touche pas le bouton de commande 13, de sorte que l'arbre 6 entraîné par la turbine 5 peut tourner à grande vitesse sans frottement.

Lorsque le dentiste veut changer d'outil, il presse du doigt le bouton 13 contre la force du ressort 14, ce qui pousse en avant le poussoir 22 et la pince 20. L'encliquetage du ressort 28 dans l'arbre 6 oppose alors une force axiale résistante supérieure à 22 N, par exemple 25 N ou plus, jusqu'à ce que le dentiste surmonte cette force et fasse ainsi avancer la pince, afin que les extrémités des mâchoires 31 glissent sur les surfaces obliques 44 de la douille de serrage 19 et soient écartées, ce qui libère l'outil. L'inclinaison des surfaces obliques 34 et 44 de la pince et de la douille peut-être choisie de façon que la pression axiale à exercer à ce moment-là par le dentiste soit relativement faible, pour des raisons de confort d'utilisation. Le dentiste ne doit donc exercer une force relativement importante qu'au tout début de l'opération, pour produire le déclic initial.

La pince 20 étant ainsi maintenue ouverte, le dentiste peut y introduire la queue 16 d'un nouvel outil, qui va buter contre le poussoir 22 et repousser celui-ci et la pince vers le haut jusqu'à ce que le ressort 28 s'encliquette de nouveau dans la gorge de l'arbre 6 pour maintenir la pince en position de service.

La figure 7 représente une variante de la douille de desserrage 19 illustrée par les figures 3 et 4. Les surfaces obliques coniques 43 et 44 sont remplacées ici par des paires opposées de surfaces obliques planes 63 et 64, avec un arrondi cylindrique 65 entre elles. Cet agencement fonctionne de la même manière que l'exemple précédent, mais offre un contact linéaire plus étendu entre les mâchoires de la pince et la douille de desserrage.

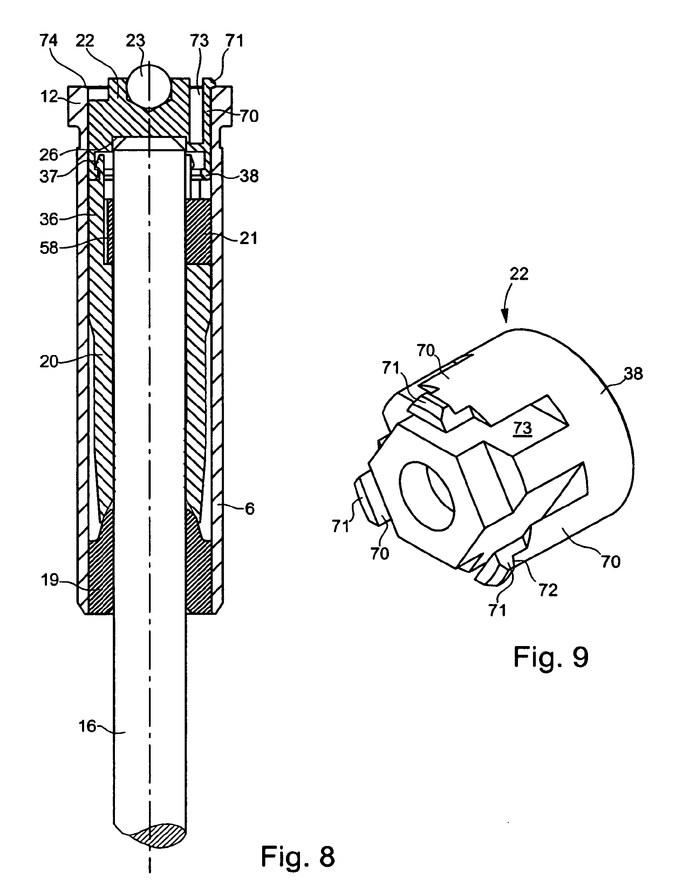

Les figures 8 et 9 montrent un autre mode de réalisation du poussoir 22 et de son dispositif de retenue à déclic sur l'arbre 6. Pour remplacer l'accrochage élastique du poussoir 22 par encliquetage du ressort 28 représenté à la figure 1, le poussoir est muni de bras longitudinaux flexibles 70, ici au nombre de trois, se terminant chacun par un crochet 71 capable de s'accrocher au bord 74 de l'extrémité arrière 12 de l'arbre 6, grâce à une surface oblique 72 du crochet. Chaque bras 70 est formé par l'exécution d'une entaille 73 le séparant du corps central du poussoir. L'angle d'inclinaison de la surface oblique 72 et les caractéristiques élastiques du bras 70 déterminent une force limite d'accrochage, qui doit être supérieure à la force minimale de l'arrachage de l'outil comme on l'a expliqué précédemment. Dès que l'opérateur surmonte cette force en pressant le bouton 13 pour ouvrir la pince, un déclic se produit par diminution de la force axiale résistante et l'opérateur ne doit plus surmonter que la force exercée sur la pince 20 par la douille de desserrage 19. Comme dans l'exemple précédent, les bras flexibles 36 de la pince 20 passent à travers les canaux 58 du canon de guidage arrière 21, assurant l'entraînement en rotation de la pince, et s'accrochent par leur extrémité profilée 37 au rebord 38 du poussoir 22.

D'autres moyens de retenue à déclic peuvent être prévus à la place de ceux qui sont représentés dans les dessins. Par exemple la pince 20 pourrait avoir à l'arrière seulement deux bras élastiques tels que les bras 36, pour s'accrocher au poussoir 22, et deux autres bras élastiques s'accrochant à un bord du canon de guidage arrière 21 ou à des bords de certains orifices de l'arbre 6 pour assurer une retenue longitudinale à déclic.

Parmi d'autres variantes possibles, on mentionnera aussi que le canon de guidage arrière 21 pourrait être supprimé dans certains cas, sa fonction étant reprise par la partie annulaire 30 de la pince ou par le poussoir 22, avec une liaison à clavette avec l'arbre 6 pour assurer l'entraînement en rotation. D'autre part, le poussoir peut être soudé à la pince dans tous les cas où leur assemblage n'a pas besoin d'être démontable.

## Revendications

1. Pièce à main à usage dentaire ou chirurgical, comportant un arbre creux (6) associé à des moyens d'entraînement en rotation (5) et dans lequel sont montés une pince (20) pour le serrage de la queue (16) d'un outil amovible, une douille de desserrage (19) et un poussoir coulissant (22) placé dans une extrémité arrière de l'arbre creux et commandé par un bouton manuel (13) pour rapprocher mutuellement la pince et la douille de desserrage, la pince comportant une partie annulaire (30) et des mâchoires (31) agencées pour serrer par élasticité la queue de l'outil dans une position de service, ces mâchoires s'étendant longitudinalement à partir de la partie annulaire en direction de la douille de desserrage (19), laquelle comporte des surfaces extérieures obliques (44, 64) capables de s'appuyer contre des extrémités libres des mâchoires pour les écarter,
**caractérisée en ce que** la douille de desserrage (19) est montée dans une extrémité avant de l'arbre creux (6), c'est-à-dire du côté de l'outil, et **en ce que** la pince (20) est montée de manière coulissante dans l'arbre, ses mâchoires (31) étant dirigées vers l'avant, la pince étant liée longitudinalement au poussoir (22), lequel permet à un opérateur de déplacer la pince en direction de l'avant pour l'appuyer contre la douille de desserrage.

2. Pièce à main selon la revendication 1, **caractérisée en ce que** la douille de desserrage (19) est fixe dans l'arbre creux et forme un canon de guidage avant pour la queue de l'outil.

3. Pièce à main selon la revendication 1, **caractérisée en ce que** le poussoir (22) comporte une paroi transversale (26) formant une butée axiale pour la queue de l'outil.

4. Pièce à main selon l'une des revendications précédentes, **caractérisée en ce qu**'un canon de guidage arrière (21), pourvu d'un alésage cylindrique (54) pour recevoir la queue de l'outil, est monté de manière fixe dans l'arbre creux entre la partie annulaire (30) de la pince et la paroi transversale (26) du poussoir de commande et comporte des canaux (58) pour des bras longitudinaux (36) reliant le poussoir à la pince.

5. Pièce à main selon la revendication 4, **caractérisée en ce qu**'au moins un desdits bras longitudinaux (36) est élastique radialement et assure une liaison par encliquetage entre la pince et le poussoir.

6. Pièce à main selon la revendication 1, **caractérisée en ce que** l'ensemble formé par la pince (20) et le poussoir (22) est pourvu d'un dispositif de retenue à déclic, agencé pour opposer une résistance initiale prédéterminée à un déplacement de cet ensemble vers l'avant à partir de la position de service.

7. Pièce à main selon la revendication 6, **caractérisée en ce que** le dispositif de retenue à déclic comporte des moyens d'accrochage élastiques (28,70, 71) qui prennent appui sur l'arbre creux.

8. Pièce à main selon la revendication 7, **caractérisée en ce que** lesdits moyens d'accrochage élastiques sont formés par un ressort (28) disposé sur le pourtour du poussoir (22) et coopérant avec une gorge intérieure de l'arbre creux.

9. Pièce à main selon la revendication 7, **caractérisée en ce que** lesdits moyens d'accrochage élastiques sont ménagés sur des bras longitudinaux élastiques (70) de la pince ou du poussoir de commande et s'accrochent à au moins un bord (74) de l'arbre creux.

## Claims

1. Hand held piece for dental or surgical use, comprising a hollow shaft (6) associated with means for driving in rotation (5), and in which are mounted a clamp (20) for gripping the shank (16) of a removable tool, a loosening sleeve (19) and a sliding pusher (22) placed in a rear end of the hollow shaft and controlled by a manual button (13) for moving the clamp and loosening sleeve towards each other, the clamp including an annular part (30) and jaws (31) arranged for gripping the tool shank via resilience in a working position, said jaws extending longitudinally from the annular part towards the loosening sleeve (19), which includes external oblique surfaces (44, 64) capable of abutting against the free ends of the jaws to move said jaws apart,
**characterized in that** the loosening sleeve (19) is mounted in a front end of the hollow shaft (6), i.e. on the tool side, and **in that** the clamp (20) is slideably mounted in the shaft, the jaws thereof (31) being directed forwards, the clamp being longitudinally connected to the pusher (22), which enables an operator to move the clamp forwards to abut against the loosening sleeve.

2. Hand held piece according to claim 1, **characterized in that** the loosening sleeve (19) is fixed in the hollow shaft and forms a front guide bush to the tool shank.

3. Hand held piece according to claim 1, **characterized in that** the pusher (22) includes a transverse wall (26) forming an axial stop member for the tool shank.

4. Hand held piece according to any of the preceding claims, **characterized in that** a back guide bush (21), provided with a cylindrical bore (54) for receiving the tool shank, is fixedly mounted in the hollow shaft between the annular part (30) of the clamp and the transverse wall (26) of the control pusher and includes channels (58) for longitudinal arms (36) connecting the pusher to the clamp.

5. Hand held piece according to claim 4, **characterized in that** at least one of said longitudinal arms (36) is resilient radially and provides a snap fit connection between the clamp and the pusher.

6. Hand held piece according to claim 1, **characterized in that** the assembly formed by the clamp (20) and the pusher (22) is provided with a snap fit hold device, arranged for offering a predetermined initial resistance to a movement of said assembly forwards from the working position.

7. Hand held piece according to claim 6, **characterized in that** the snap fit holding device includes resilient hooking means (28, 70, 71) carried on the hollow shaft.

8. Hand held piece according to claim 7, **characterized in that** said resilient hooking means are formed by a spring (28) arranged on the periphery of the pusher (22) and cooperating with an inner groove of the hollow shaft.

9. Hand held piece according to claim 7, **characterized in that** said resilient hooking means are arranged on resilient longitudinal arms (70) of the clamp or the control pusher and hook onto at least one edge (74) of the hollow shaft.

## Patentansprüche

1. Handteil für zahnärztliche oder chirurgische Nutzung, mit einer Hohlwelle (6), der Drehantriebsmittel (5) zugeordnet sind und in der eine Zange (20) zum Festklemmen des hinteren Endes (16) eines entnehmbaren Werkzeugs, eine Lösehülse (19) und ein Gleitdrücker (22), der in einem hinteren Ende der Hohlwelle angeordnet und durch einen manuellen Knopf (13) gesteuert wird, um die Zange und die Lösehülse einander anzunähern, angebracht sind, wobei die Zange einen ringförmigen Teil (30) und Klemmbacken (31), die angeordnet sind, um durch Elastizität das hintere Ende des Werkzeugs in einer Arbeitsposition einzuklemmen, umfasst, wobei sich diese Klemmbacken longitudinal ausgehend von dem ringförmigen Teil in Richtung der Lösehülse (19) erstrecken, die ihrerseits schräge äußere Oberflächen (44, 64) aufweist, die sich an den freien Enden der Klemmbacken abstützen können, um sie voneinander zu beabstanden,
**dadurch gekennzeichnet, dass** die Lösehülse (19) in einem vorderen Ende der Hohlwelle (6), d. h. auf Seiten des Werkzeugs, angebracht ist und dass die Zange (20) gleitend in der Welle angebracht ist, wobei ihre Klemmbacken (31) nach vorn gerichtet sind, wobei die Zange longitudinal mit dem Drücker (22) verbunden ist, was einer Bedienungsperson ermöglicht, die Zange in Richtung nach vorn zu verlagern, um sie an der Lösehülse abzustützen.

2. Handteil nach Anspruch 1, **dadurch gekennzeichnet, dass** die Lösehülse (19) in der Hohlwelle befestigt ist und ein vorderes Führungsrohr für das hintere Ende des Werkzeugs bildet.

3. Handteil nach Anspruch 1, **dadurch gekennzeichnet, dass** der Drücker (22) eine Querwand (26) aufweist, die einen axialen Anschlag für das hintere Ende des Werkzeugs bildet.

4. Handteil nach einem der vorhergehenden Ansprüche, **dadurch gekennzeichnet, dass** ein hinteres Führungsrohr (21), das mit einer zylindrischen Bohrung (54) versehen ist, um das hintere Ende des Werkzeugs aufzunehmen, in der Hohlwelle zwischen dem ringförmigen Teil (30) der Zange und der transversalen Wand (26) des Steuerdrückers fest angebracht ist und Kanäle (58) für die longitudinalen Arme (36), die den Drücker mit der Zange verbinden, enthält.

5. Handteil nach Anspruch 4, **dadurch gekennzeichnet, dass** wenigstens einer der longitudinalen Arme (36) radial elastisch ist und eine Verbindung durch Einrasten zwischen der Zange und dem Drücker gewährleistet.

6. Handteil nach Anspruch 1, **dadurch gekennzeichnet, dass** die durch die Zange (20) und den Drücker (22) gebildete Gesamtheit mit einer Haltevorrichtung mit Auslöseklinke versehen ist, der ausgelegt ist, um einer Verlagerung dieser Gesamtheit nach vorn ausgehend von der Arbeitsposition einen vorbestimmten anfänglichen Widerstand entgegenzusetzen.

7. Handteil nach Anspruch 6, **dadurch gekennzeichnet, dass** die Haltevorrichtung mit Auslöseklinke elastische Einhakmittel (28, 70, 71) aufweist, die sich an der Hohlwelle abstützen.

8. Handteil nach Anspruch 7, **dadurch gekennzeichnet, dass** die elastischen Einhakmittel durch eine Feder (28) gebildet sind, die an dem Umfang des Drückers (22) angeordnet ist und mit einer Innennut der Hohlwelle zusammenwirkt.

9. Handteil nach Anspruch 7, **dadurch gekennzeichnet, dass** die elastischen Einhakmittel an den elastischen longitudinalen Armen (70) der Zange oder des Steuerdrückers ausgebildet sind und in wenigstens einen Rand (74) der Hohlwelle eingehakt sind.
